# EUROPÄISCHE PATENTANMELDUNG

(11) **EP 3 957 642 A1**
(43) Veröffentlichungstag der Anmeldung: **23.02.2022**
(21) Anmeldenummer: 20191435.5
(22) Anmeldetag: 18.08.2020
(51) Int. Cl.: C07F 9/6574, B01J 31/24, C07C 45/50, C07F 15/00

(54) **6,6'-([1,1'-BIPHENYL]-2,3'-DIYLBIS(OXY))DIDIBENZO[D,F][1,3,2]DIOXAPHOSPHEPINE**

(71) Anmelder: Evonik Operations GmbH, 45128 Essen (DE)
(72) Erfinder: FRANKE, Robert, 45772 Marl (DE); BÖRNER, Armin, 18059 Rostock (DE); KLOSS, Svenja, 72461 Albstadt (DE); SELENT, Detlef, 18059 Rostock (DE)
(74) Vertreter: Evonik Patent Association

(57) **Zusammenfassung**

6,6'-([1,1'-Biphenyl]-2,3'-diylbis(oxy))didibenzo[d,f][1,3,2]dioxaphosphepine und deren Verwendung in der Hydroformylierung.

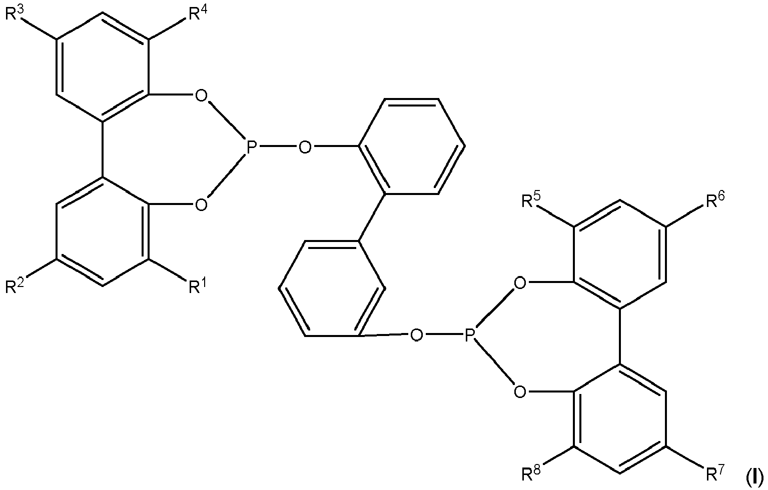

## Beschreibung

Die Erfindung betrifft 6,6'-([1,1'-Biphenyl]-2,3'-diylbis(oxy))didibenzo[d,f][1,3,2]dioxaphosphepine und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In EP 0 577 042 A1 ist auf Seite 6 der folgende Ligand dargestellt:

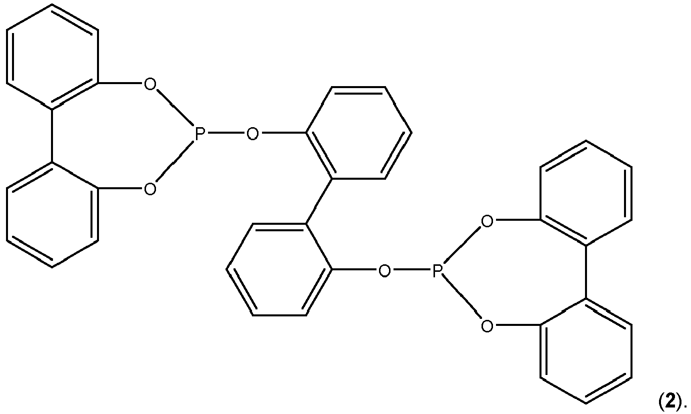

Die technische Aufgabe der Erfindung ist die Bereitstellung neuer Liganden, welche in der Hydroformylierung von Olefinen eine gesteigerte n/iso-Selektivität verglichen zu dem aus dem Stand der Technik bekennten Liganden aufweisen.

Die Aufgabe wird gelöst durch eine Verbindung gemäß Anspruch 1.

Verbindung gemäß der Struktur (**I**): 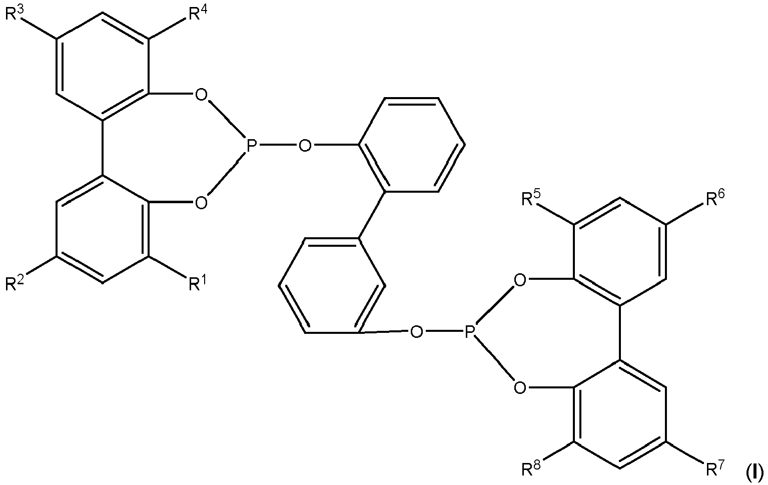 wobei R¹, R², R³, R⁴, R⁵, R⁶, R⁷, R⁸ ausgewählt sind aus: -H, -(C₁-C₁₂)-Alkyl, -O-(C₁-C₁₂)-Alkyl.

In einer Ausführungsform sind R¹, R⁴, R⁵, R⁸ ausgewählt aus: -H, -(C₁-C₁₂)-Alkyl.

In einer Ausführungsform stehet mindestens einer der Reste R¹, R⁴, R⁵, R⁸ für -H.

In einer Ausführungsform stehen R¹, R⁴, R⁵, R⁸ für -H.

In einer Ausführungsform sind R², R³, R⁶, R⁷ ausgewählt aus: -H, -O-(C₁-C₁₂)-Alkyl.

In einer Ausführungsform stehen mindestens einer der Reste R², R³, R⁶, R⁷ für -H.

In einer Ausführungsform stehen R², R³, R⁶, R⁷ für -H.

In einer Ausführungsform weist die Verbindung die Struktur **(1)** auf:

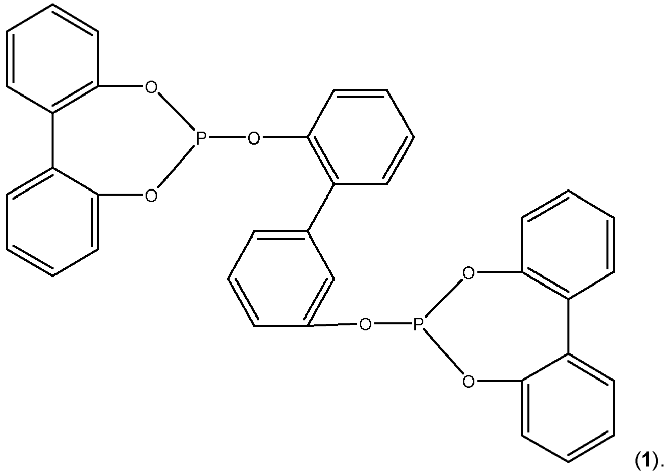

Neben der Verbindung als solche wird auch deren Verwendung zur Katalyse einer Hydroformylierungsreaktion beansprucht.
Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 10 bis 60 bar.

Besonders bevorzugt sind eine Temperatur von 100 °C bis 140 °C und ein Druck von 20 bis 50 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H ^{∗} (BF³¹P / BF¹H) = SR¹H ^{∗} 0,4048.

### Synthese 6,6'-([1,1'-Biphenyl]-2,3'-diylbis(oxy))didibenzo[d,f][1,3,2]dioxaphosphepin (1)

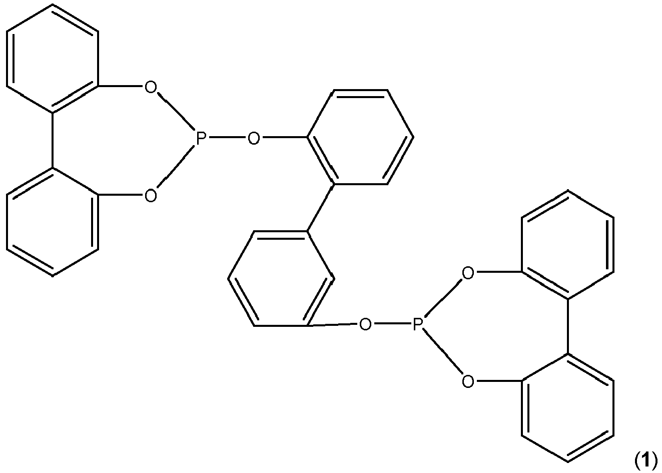

Eine Lösung aus 0,10 g 2,3'-Biphenol (0,56 mmol) und 0,3 ml Triethylamin (2,24 mmol) in 4 ml THF wurde bei -20 °C zu einer gerührten Lösung aus 0,39 g 6-Chloro-dibenzo[*d,f*[1,3,2]-dioxaphosphepin (1,56 mmol) in 4 ml THF getropft. Über Nacht wurde die Lösung weiter gerührt und auf Raumtemperatur erwärmt. Das Lösungsmittel wurde anschließend im Vakuum entfernt, der Rückstand in 10 ml Toluol aufgenommen und durch eine G4-Fritte filtriert. Danach wurde das Lösungsmittel des Filtrats im Vakuum entfernt. Das zurückbleibende gelbe Öl wurde durch Säulenchromatographie (Eluentengemisch Dichlormethan/n-Heptan = 3:7) aufgearbeitet. Es wurden 0,10 g eines weißen Feststoffes erhalten (Ausbeute: 30 %).
¹H-NMR (300 MHz, CD₂Cl₂): δ (ppm) = 7,39-7,53 (m; 6H); 7,18-7,39 (m; 16H); 7,00-7,05 (m; 2H). ¹³C-NMR (75 MHz, CD₂Cl₂): δ (ppm) = 151,8 (d; *J*_{CP} = 7,9 Hz); 149,2 (d; *J*_{CP} = 5,0 Hz); 149,2 (d; *J*_{CP} = 5,0 Hz); 149,0 (d; *J*_{CP} = 7,6 Hz; *C*_{Ar}OP); 139,9; (d; *J*_{CP} = 3,2 Hz); 133,3; 131,5; 131,4 (d; *J*_{CP} = 3,3 Hz); 131,3 (d; *J*_{CP} = 3,3 Hz); 130,3; 130,2; 129,9; 129,6; 129,4; 126,3; 125,8; 125,8; 125,1; 122,4; 122,3; 121,4; 121,2; 119,8; 119,6.
³¹P-NMR (121 MHz, CD₂Cl₂): δ (ppm) = 144,3 (s); 144,0 (s).
HRMS (ESI): Berechnet für C₃₆H₂₄O₆P₂ (M+H⁺) 615,11209, gefunden 615,11174. Berechnet für C₃₆H₂₄O₆P₂ (M+Na⁺) 637,09403, gefunden 637,09386.

### Synthese 2,2'-Bis(dibenzo[d,f][1,3,2]dioxaphosphepin-6 yloxy)-1,1'-biphenyl (2) (Vergleichsligand)

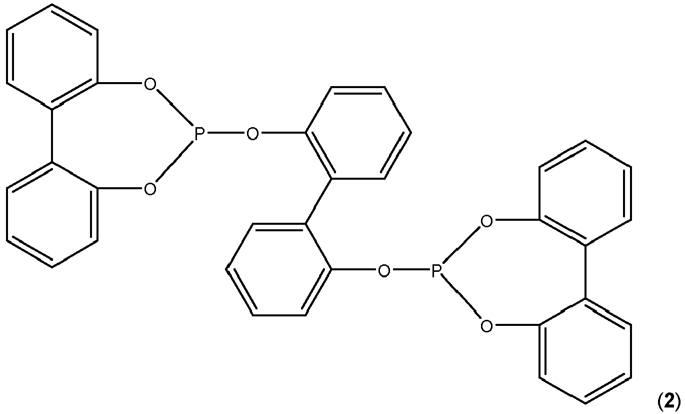

Eine Lösung aus 0,99 g 2,2'-Biphenol (5,29 mmol) und 3 ml Triethylamin (21,2 mmol) in 7 ml THF wurde bei -20 °C zu einer gerührten Lösung aus 2,65 g 6-Chloro-dibenzo[*d,f*[1,3,2]-dioxaphosphepin (10,59 mmol) in 7 ml THF getropft. Über Nacht wurde die Lösung weiter bei Raumtemperatur gerührt. Das Lösungsmittel wurde anschließend im Vakuum entfernt und der Rückstand in 15 ml Toluol aufgenommen. Die trübe Lösung wurde durch eine G4-Fritte filtriert und die Lösungsmittelreste im Filtrat anschließend im Vakuum entfernt. Der ölige Rückstand wurde in einer geringen Menge Dichlormethan (ca. 3 ml) gelöst. Anschließend wurde unter Rühren *n-*Heptan hinzugegeben, bis sich die Lösung trübte. Über Nacht wurde die Lösung im Kühlschrank aufbewahrt und die überstehende klare Lösung am nächsten Tag dekantiert und der Feststoff im Vakuum getrocknet. Es wurden 1,09 g eines gelblich-weißen Feststoffs erhalten (Ausbeute: 34 %). ¹H-NMR (300 MHz, CD₂Cl₂): δ (ppm) = 7,30-7,49 (m; 11H); 7,23-7,29 (m; 9H); 6,85-6,94 (m; 4H). ¹³C-NMR (75 MHz, CD₂Cl₂): δ (ppm) = 149,9 (m; *C*_{Ar}OP); 149,2 (m; *C*_{Ar}OP); 132,5; 131,3; 130,6; 130,0; 129,5; 129,4; 125,6; 124,6; 122,3; 120,8 (m).
³¹P-NMR (121 MHz, CD₂Cl₂): δ (ppm) = 144,7 (s).
HRMS (ESI): Berechnet für C₃₆H₂₄O₆P₂ (M+H)⁺ 615,11209, gefunden 615,11203. Berechnet für C₃₆H₂₄O₆P₂ (M+Na)⁺ 637,09403, gefunden 637,09394.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das als Substrat eingesetzte n-Octen (Oxeno GmbH, Octenisomerengemisch aus 1-Octen: 3 %; cis+trans-2-Octen: 49 %; cis+trans-3-Octen: 29 %; cis+trans-4-Octen: 16 %; gerüstisomere Octene: 3 %) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 0,0021 g Rh(acac)(CO)₂ und die entsprechende Menge an Phosphitverbindung eingewogen und mit 8,0 ml Toluol aufgefüllt. Die jeweils eingebrachte Masse an Toluol wurde für die GC-Analyse bestimmt. Anschließend wurden 1,80 g n-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %) : CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 20 bar erhöht und die Reaktion bei konstantem Druck für 4 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m x 0,2 mm x 0,5 µm. Die quantitative Bestimmung von Restolefin und Aldehyd erfolgte gegen das Lösungsmittel Toluol als interner Standard.

### Ergebnisse der Katalyseversuche

### Reaktionsbedingungen 1:

[Rh]: 1,0^{∗}10⁻³ mol/l, L:Rh = 1:2, p: 20 bar, T: 120 °C; t: 4 h

**Tabelle 1: Hydroformylierung der n-Octene**

| Ligand | n/iso-Selektivität in % |
|---|---|
| **1*** | 61,6 |
| **2** | 38,9 |

| | |
|---|---|
| * erfindungsgemäße Verbindung | |

### Reaktionsbedingungen 2:

[Rh]: 1,0^{∗}10⁻³ mol/l, L:Rh = 1:4, p: 20 bar, T: 120 °C; t: 4 h

**Tabelle 2: Hydroformylierung der n-Octene**

| Ligand | n/iso-Selektivität in % |
|---|---|
| **1*** | 78,3 |
| **2** | 68,3 |

| | |
|---|---|
| * erfindungsgemäße Verbindung | |

### Definition der Selektivität:

Bei der Hydroformylierung gibt es die n/iso-Selektivität: das Verhältnis von linearem Aldehyd (= n) zu verzweigtem Aldehyd (= iso). Hierbei bedeutet die Selektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Mit der erfindungsgemäßen Verbindung **(1)** konnte eine gegenüber dem Vergleichsliganden **(2)** gesteigerte Selektivität erzielt werden.

Die durchgeführten Versuche belegen, dass die gestellte Aufgabe durch die erfindungsgemäße Verbindung gelöst wird.

## Patentansprüche

1. Verbindung gemäß der Struktur (**I**): 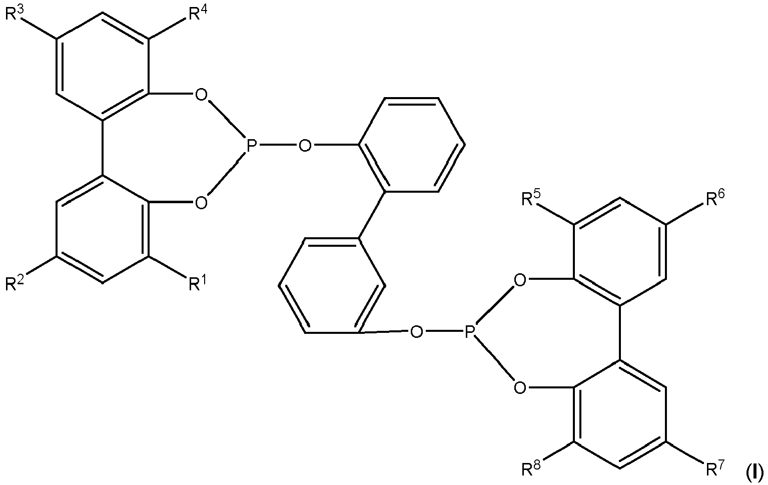 wobei R¹, R², R³, R⁴, R⁵, R⁶, R⁷, R⁸ ausgewählt sind aus: -H, -(C₁-C₁₂)-Alkyl, -O-(C₁-C₁₂)-Alkyl.

2. Verbindung nach Anspruch 1,
wobei R¹, R⁴, R⁵, R⁸ ausgewählt sind aus: -H, -(C₁-C₁₂)-Alkyl.

3. Verbindung nach einem der Ansprüche 1 oder 2,
wobei mindestens einer der Reste R¹, R⁴, R⁵, R⁸ für -H stehet.

4. Verbindung nach einem der Ansprüche 1 bis 3,
wobei R¹, R⁴, R⁵, R⁸ für -H stehen.

5. Verbindung nach einem der Ansprüche 1 bis 4,
wobei R², R³, R⁶, R⁷ ausgewählt sind aus: -H, -O-(C₁-C₁₂)-Alkyl.

6. Verbindung nach einem der Ansprüche 1 bis 5,
wobei mindestens einer der Reste R², R³, R⁶, R⁷ für -H steht.

7. Verbindung nach einem der Ansprüche 1 bis 6,
wobei R², R³, R⁶, R⁷ für -H stehen.

8. Verbindung nach einem der Ansprüche 1 bis 7,
wobei die Verbindung die Struktur (1) aufweist:

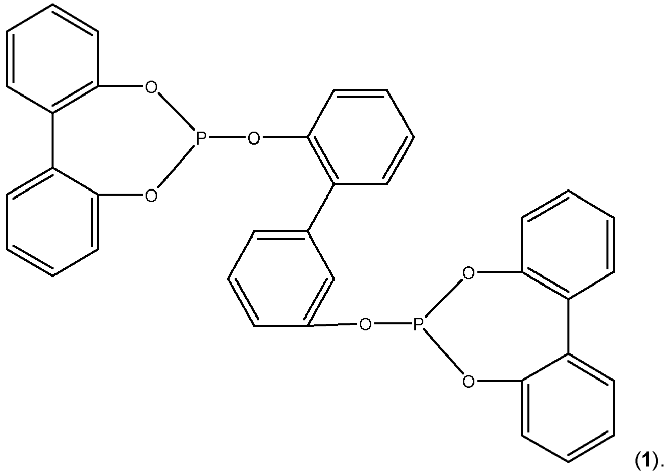

9. Verwendung einer Verbindung nach einem der Ansprüche 1 bis 8,
in einem Ligand-Metall-Komplex zur Katalyse einer Hydroformylierungsreaktion.

10. Verfahren umfassend die Verfahrensschritte:
a) Vorlegen eines Olefins,
b) Zugabe einer Verbindung nach einem der Ansprüche 1 bis 8 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.
